(19) Europäisches Patentamt
European Patent Office
Office européen des brevets

(11) Numéro de publication : **0 453 356 A2**

(12) **DEMANDE DE BREVET EUROPEEN**

(21) Numéro de dépôt : **91400988.1**

(51) Int. Cl.$^5$ : **C07C 51/235**, C07B 33/00

(22) Date de dépôt : **15.04.91**

(30) Priorité : **20.04.90 FR 9005067**

(43) Date de publication de la demande : **23.10.91 Bulletin 91/43**

(84) Etats contractants désignés : **AT BE CH DE FR GB IT LI LU NL**

(71) Demandeur : **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cédex 07 (FR)**

(72) Inventeur : **Campo, Philippe**
**13, rue Carnac**
**F-78180 Montigny-le-Bretonneux (FR)**
Inventeur : **Petrignani, Jean-François,**
**Résidence du Chevalier**
**d'Orsay, Bâtiment les Doukas, 99, rue de Paris**
**F-91400 Orsay (FR)**
Inventeur : **Cocolios, Panayotis**
**44, rue de Marcoussis**
**F-91470 Limours (FR)**
Inventeur : **Ledon, Henry**
**1 bis, avenue de l'Assemblée Nationale**
**F-78000 Versailles (FR)**

(74) Mandataire : **Caen, Thierry Alain et al**
**L'Air Liquide, Société anonyme pour l'étude et l'exploitation des procédés Georges Claude,**
**75, quai d'Orsay**
**F-75321 Paris Cédex 07 (FR)**

(54) **Procédé d'oxydation de composés organiques.**

(57) L'invention concerne un procédé d'oxydation de composés organiques dans lequel on soumet un composé organique oxydable à l'action de dioxygène, dans un solvant, sous pression atmosphérique, en présence d'un système catalytique comprenant un complexe ou un sel métallique et, le cas échéant, un composé capable de solubiliser le sel métallique. Les composés oxydés obtenus sont utilisables comme intermédiaires de synthèse particulièrement dans l'industrie cosmétique, agrochimique et pharmaceutique.

**EP 0 453 356 A2**

Jouve, 18, rue Saint-Denis, 75001 PARIS

L'invention a pour objet un procédé d'oxydation sélective de composés organiques, plus spécialement un procédé d'oxydation d'aldéhydes en acides carboxyliques.

On sait que les acides carboxyliques sont des produits industriels de grand intérêt. Ils constituent entre autres des intermédiaires de synthèse particulièrement précieux dans l'industrie cosmétique, agrochimique et pharmaceutique. Ainsi, l'acide hydratropique est un intermédiaire de synthèse important en chimie fine. Nombre d'entre eux sont dotés de propriétés biologiques, tel l'acide (isobutyl-4- phényl)-2-propionique connu sous le nom générique ibuprofen utilisé comme agent anti-inflammatoire non stéroïdien. Les acides provenant de l'oxydation des aldéhydes aliphatiques linéaires à longues chaînes, de plus de 15 atomes de carbone, sont des additifs utilisables en cosmétologie.

D'une manière générale, les acides carboxyliques sont obtenus à partir des aldéhydes correspondants par oxydation stoechiométrique à l'aide d'agents d'oxydation tels que $KMnO_4$, $Ag_2O$, $CrO_3$, $MnO_2$, $H_2O_2$, catalysée ou non par $SeO_2$ ,ou par oxydation catalytique à l'aide d'oxygène (explicité dans la suite du texte par les termes dioxygène ou oxygène moléculaire).

La première méthode présente l'inconvénient d'utiliser des quantités stoechiométriques et souvent de larges excès d'oxydant par rapport à l'aldéhyde.

Dans certains cas pour des raisons de toxicité, il est nécessaire de prévoir une élimination complète de toute trace de métal par exemple le sélénium dans les acides carboxyliques obtenus compte tenu des applications envisagées. Ceci nécessite une ou plusieurs étapes de purification obérant considérablement l'économie du procédé.

En présence d'oxygène, les aldéhydes subissent une autoxydation radicalaire et ce, même à température ambiante; cette réaction s'effectue cependant de manière peu sélective. Ainsi, dans le cas de l'oxydation d'aryl-2-propionaldéhydes, on obtient simultanément des acétophénones, des aryl-1-éthanols et des formiates d'aryl-2-éthane.

Une solution partielle à ce problème a été donnée dans le brevet EP 0226566 qui rapporte l'oxydation d'aryl-2-propanaldéhydes en acides à l'aide d'oxygène moléculaire sous pression élevée, dans un solvant non polaire, à des températures voisines de 10°C et en présence de complexes solubles de manganèse ou de cobalt. Ce procédé permet de supprimer la formation de formiates, mais le mélange réactionnel contient des cétones et des alcools comme sous-produits.

On mesurera toutefois que la réalisation de la réaction d'oxydation sous pression représente un facteur de risque lié à la composition du ciel gazeux du réacteur susceptible d'être dans les limites d'inflammabilité.

Les inventeurs ont réussi à éliminer ce risque en réalisant l'oxydation dans un solvant dépourvu de point éclair et permettant de plus d'opérer à basse température, à savoir inférieure à 0°C.

L'invention a donc pour but de fournir un nouveau procédé d'oxydation à l'aide de dioxygène permettant d'obtenir des composés organiques oxydés avec des conversions et des sélectivités élevées, dans des conditions de sécurité assurée.

Elle vise plus spécialement à fournir un procédé d'obtention d'acides carboxyliques à partir des aldéhydes correspondants.

Le procédé d'oxydation de l'invention est caractérisé en ce qu'on soumet à l'action de l'oxygène moléculaire un composé organique oxydable, en opérant dans un solvant exempt d'alcool, en présence d'un système catalytique comprenant un complexe ou un sel métallique et, le cas échéant, un composé capable de faciliter la solubilisation du sel métallique par coordination.

Ces dispositions permettent de mettre à profit Le caractère oxydant du dioxygène sous des pressions variant de $10^2$ à $10^4$ KPa de préférence à $10^2$ KPa et ce, dans des conditions de sécurité absolue. Sauf indication contraire, toutes les concentrations décrites ci-dessous sont exprimées en moles.

La mise en oeuvre de basses températures influe positivement sur la sélectivité en composés oxydés. De ce fait, on opère de préférence à des températures comprises entre + 20° et - 50°C, plus préférentiellement comprises entre 0 et - 20°C.

On observera que la réaction d'oxydation est réalisée dans un solvant très peu, voire non inflammable.

Les solvants mis en oeuvre sont avantageusement liquides aux températures de + 20°C à - 50°C, ne présentent pas de point éclair et sont capables de solubiliser l'oxygène.

Les solvants particulièrement appropriés sont des hydrocarbures halogénés, dont certains sont, par exemple, commercialisés sous la marques Fréon$^R$ ou la marque Foranne$^R$. Il s'agit d'hydrocarbures partiellement ou totalement substitués par du chlore et/ou du fluor.

Parmi ces Fréons$^R$, on citera le Fréon-11 ou trichlorofluorométhane de formule $CC1_3F$ (appelé F-11), le F-12 qui correspond au dichloro-difluorométhane $CC1_2F_2$, le F-13 ou monochlorotrifluorométhane $CC1F_3$, le F-14 ou tétrafluorométhane $CF_4$, le F-21 ou dichlorofluorométhane $CHC1_2F$, le F-22 ou monochlorodifluorométhane $CHC1F_2$, le F-23 ou fluoroforme $CHF_3$, le F-112 ou tétrachloro-1,1,2,2 fluoro 1,2-éthane $CC1_2F$-$CC1_2F$, le F-113 ou trichloro 1,1,2 trifluoro-1,2,2- éthane, $CC1_2F$-$CC1F_2$, le F-114 ou dichloro-1,2

tétrafluoroéthane, $CC1F_2$-$CC1F_2$, le F-115 ou chloro-1, pentafluoro-1,1,2,2,2-éthane, $CC1F_2$-$CF_3$ ou encore le F-142 chloro-1 difluoro-1,1 éthane, $CC1F_2CH_3$.

Le Fréon$^R$-11 présente l'avantage d'assurer une solubilité satisfaisante de l'oxygène et de conduire à un rendement en composé oxydé de l'ordre de 90%.

De même, le Fréon$^R$-113 conduit à des résultats particulièrement satisfaisants. Ses caractéristiques sont les suivantes :

Péb: 47, 6 ° C/1 atm., $P_f$: -36, 4 ° C, μ (moment bipolaire) = 0; solubilité $O_2$ = 2,5 x $10^{-2}$M à 20°C, pas de point éclair. D'autres Fréons$^R$ appropriés comprennent le Fréon- 21 et le Fréon-114.

Le solvant halogénocarboné mis en oeuvre peut être additionné si on le souhaite, notamment pour favoriser la dissolution du catalyseur, d'un solvant miscible dans lequel le complexe ou le sel métallique est fortement soluble par coordination. Des molécules de ce dernier solvant jouent alors le rôle de ligand(s) additionnel(s).

Le système catalytique utilisé comprend un composé métallique avantageusement constitué par un complexe ou un sel métallique constitué avantageusement par un sel de métal de transition. Les complexes sont constitués d'un cation métallique, choisi plus particulièrement parmi le manganèse ou le cobalt, et d'un ligand organique appartenant à la famille des isoindolines ou des salicylidèneimines. Les sels sont des nitrates, des sulfates, des carbonates, des halogénures et plus spécialement des acétates de manganèse ou de cobalt. Le catalyseur peut être immobilisé. Comme support inerte approprié, on citera $SiO_2$, $Al_2O_3$, MgO, ZnO, $TiO_2$, terre de diatomées, argile ou charbon activé. Le catalyseur se trouve alors dispersé dans la solution par agitation et pourra être éliminé par simple filtration à la fin de la réaction. Cette disposition permet à la fois de supprimer les problèmes de pollution des produits obtenus par les traces des métaux et de réutiliser, si on le souhaite, le catalyseur.

En vue notamment de faciliter la solubilisation du sel métallique, on utilise une base hétérocyclique azotée, soluble dans le solvant utilisé, aux températures mises en oeuvre, capable de coordiner le métal de transition.

Une base hétérocyclique azotée particulièrement appropriée comme ligand est constituée par la pyridine.

D'autres bases utilisables comprennent les alkyl pyridines, en particulier la 3,5-lutidine, la 3- et la 4-picoline et la 4-tertiobutyl-pyridine. L'utilisation de ces bases permet d'augmenter par exemple le rendement en acide carboxylique (72-77%) et la conversion en aldéhyde (97%), pour des temps de réaction de 5 heures au lieu de 7 (facteur cinétique).

En outre, l'utilisation d'un initiateur permet de réduire, voire de supprimer la période d'induction généralement observée. Les peroxydes organiques et plus spécialement les peracides organiques s'avèrent particulièrement appropriés.

Selon un mode de réalisation avantageux de l'invention, le rapport des concentrations [catalyseur]/[substrat à oxyder] est de l'ordre de 1% à 1‰ préférentiellement de 1 à 0,5% ; le rapport de concentration [hétérocycle]/[métal] est de l'ordre de 5 à 50 et de préférence compris entre 10 et 30.

Pour abaisser le rapport de concentration [catalyseur]/[substrat à oxyder] tout en conservant un rendement élevé, il est avantageux de rajouter une charge ou plusieurs charges de composé oxydable au cours de la réaction d'oxydation.

La conduite de la réaction d'oxydation selon l'invention à basse température avec les solvants du type indiqué ci-dessus permet d'apporter des améliorations substantielles tant au plan de la sélectivité que de la cinétique dans les réactions d'autoxydation par $0_2$ habituellement effectuées.

Le domaine d'application du procédé de l'invention est donc très général.

Il présente un intérêt tout particulier pour l'obtention d'acides carboxyliques aliphatiques ou aromatiques, à partir des aldéhydes correspondants.

Comme aldéhydes convenant pour la mise en oeuvre de l'invention, on citera ceux répondant à la formule suivante :

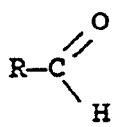

dans laquelle R représente

- un groupe hydrocarboné de 1 à 30 atomes de carbone, linéaire ou ramifié, comportant le cas échéant dans la chaîne, un ou plusieurs atomes d'oxygène et/ou un ou plusieurs groupes oxo, ce groupe hydrocarboné étant choisi parmi les groupes alcoyle, alcoxy ou acyle,
- un groupe cycloalcoyle de 1 à 30 atomes de carbone,
- un groupe aryle comprenant un ou plusieurs cycles aromatiques,

– un groupe arylacyle où les parties aryle et acyle sont respectivement telles que définies ci-dessus,

– un groupe hétérocyclique;

ces différents groupes étant le cas échéant substitués par un ou plusieurs groupements inertes dans les conditions réactionnelles, tels que des atomes d'halogène, des groupes alcoyle, alcoxy dans lesquels la partie alcoyle, linéaire ou ramifiée, comporte de 1 à 5 atomes de carbone, -COOR' avec R' représentant un groupe alcoyle de 1 à 5 atomes de carbone, aryloxy, benzo et nitro.

Dans ces aldéhydes, les groupes alcoyle comprennent notamment de 1 à 5 atomes de carbone. Les groupes aryle sont notamment choisis parmi les groupes phényle et naphtyle. Comme hétérocycle, on citera entre autres le benzoxazole.

Ces aldéhydes sont des produits commerciaux ou sont synthétisables selon les réactions classiques de la chimie organique. A cet égard, on peut avoir recours à l'oxydation d'hydrocarbures saturés ou insaturés, de dérivés monohalogénés d'hydrocarbures saturés ou aromatiques, d'alcools primaires. On peut également préparer ces aldéhydes en hydrolysant des dérivés gemdihalogénés primaires, des aldimines, en déshydratant des α-glycols. Il est possible en variante d'oxyder des α- glycols bisecondaires.

D'autres techniques largement utilisées comprennent la formylation selon le procédé GATTERMAN - KOCH, l'hydroformylation catalytique des oléfines terminales, la réduction du type ROSENMUND, la décomposition des acides α-alcools, ou encore la mise en oeuvre de la condensation de DARZENS.

L'acide carboxylique formé est avantageusement récupéré par extraction.

Dans les exemples qui suivent, on rapporte d'autres caractéristiques et avantages de l'invention.

## METHODE GENERALE

On utilise un réacteur muni d'une double enveloppe pour la circulation de liquides réfrigérants.

Après avoir conditionné à la température souhaitée et purgé le montage à l'oxygène ( $PO_2$ = 104 kPa ), on introduit successivement : le catalyseur, le ligand azoté, le solvant, le substrat, l'initiateur, puis on fait barboter du dioxygène.

L'évolution de la réaction est suivie :

– par la courbe de consommation d'oxygène en fonction du temps,

– par des prélèvements de brut réactionnel dilués dans du $CDCl_3$ et analysés en spectroscopie RMN du proton.

Lorsque le système ne consomme plus d'oxygène, on ajoute au mélange réactionnel du dichlorométhane et de l'eau. Le pH est ajusté à 11 à l'aide d'une solution aqueuse de soude à 10%. Après séparation des phases, la phase aqueuse contenant le sel de l'acide organique est acidifiée avec de l'acide chlorhydrique à 10%, pH 1. L'acide est extrait à l'aide de dichlorométhane, séché sur du sulfate de magnésium. Le solvant est récupéré après filtration et évaporation du solvant.

## I/ PREPARATION D'ACIDE PHENYL-2-PROPIONIQUE A PARTIR DE PHENYL-2-PROPANOL

### A) EFFET DU CATALYSEUR ET DE LA TEMPERATURE

On rapporte dans le tableau 1 ci-après les conditions opératoires et les principaux résultats obtenus en procédant à l'oxydation du phényl-2-propanal en solution dans du Fréon$^R$113, en présence de catalyseurs métalliques à base de Rh, Cu, Co et Mn et en faisant varier la température entre 100 et -25°C selon le solvant mis en oeuvre.

Dans ce tableau,

NR = nombre de rotations : nombre de mole d'acide formé/nombre de mole de catalyseur,

py = pyridine (pyridine/substrat = 0,33)

ini = initiateur : peracide organique (peracide/ catalyseur = 1,66).

Les catalyseurs utilisés sont désignés dans le tableau par des symboles ayant les significations suivantes :

BPICo : bis-(2-pyridyl)-isoindolinato cobalt (II) acétate

SALSMn:éthylène-bis-(5-sulfosalicylidèneiminato) manganèse (III) acétate

SAPMn : dipropylamino-bis-salicylidèneiminatomanganèse (II) acétate

$Mn(OAc)_2$ = diacétate de manganèse II.

Tableau 1

| Essai | Catal. | Temps (H) | T (°C) | Sélectivité[a] (%) Ald. | Cét. | Acide | NR | Ald./ Catal. |
|---|---|---|---|---|---|---|---|---|
| 1 | BPICo ini | 22 | -21 | 29 | 6 | 54[b] | 71 | 100 |
| 2 | SALMn py | 5.5 | +6 | 20 | 17 | 42[b] | 80 | 100 |
| 3 | SALMn py | 5.5 | -15 | 22 | 12 | 66 | 76 | 100 |
| 4 | SALMn py | 7 | -25 | 27 | 4 | 69 | 72 | 100 |
| 5 | SALMn py, ini | 8 | -25 | 20 | 5 | 75 | 78 | 100 |
| 6 | SALMn py, EtOH | 23 | -20 | 27 | 2 | 48[c] | 71 | 100 |
| 7 | SAPMn py, ini | 7 | -20 | 53 | 5 | 42 | 47 | 100 |
| 8 | $Mn(OAc)_2$ py, ini | 7 | -20 | 21 | 8 | 71 | 70 | 88 |

a : Analyses du brut réactionnel (RMN)

b : présence du formiate correspondant

c : le complément à 100 % est constitué par des composés non caractérisés.

L'examen de ce tableau montre que le phényl-2- propanal peut être oxydé par l'oxygène, en opérant dans les conditions de l'invention, en acide phényl-2 propionique, avec une conversion de 80 % et une sélectivité en produit de 94 %.

Ces résultats mettent en évidence les avantages de l'invention.

On remarquera le rôle de la température sur la sélectivité en acide comme illustré par les essais 2 et 4, respectivement à 6°C et -25°C et pour lesquels la sélectivité est de 52,5 et 94,5 respectivement.

L'addition d'un initiateur (quelques équivalents de peracide organique) par rapport au catalyseur supprime

la période d'induction. On remarquera également que l'essai 6, comparatif, montre que la présence d'alcool grève lourdement la cinétique de la réaction.

B) FACTEUR D'ECHELLE

Le tableau 2 prouve la reproductibilité des résultats précédents de l'invention. En effet, les mêmes rendements en acide sont obtenus lorsque la quantité initiale d'aldéhyde mise en oeuvre est multipliée par un facteur $3 \times 10^4$, ceci dans les conditions décrités dans l'essai 8 du Tableau 1. Ceci démontre l'applicabilité du procédé de l'invention à une échelle industrielle.

TABLEAU 2

| Aldéhyde (mole) | Rendement Acide[a] (%) |
|---|---|
| $1{,}5\ 10^{-3}$ | 64 |
| 2 | 70 |
| 15 | 60 |
| 50 | 68 |

a : essai 8, tableau 1

C) ETUDE DE L'INFLUENCE DE DIFFERENTS LIGANDS HETEROCYCLIQUES AZOTES

On donne dans le tableau 3 ci-après les résultats concernant la sélectivité et les rendements en acide par rapport à l'aldéhyde de départ ou l'oxygène consommé, obtenus en utilisant différents ligands azotés dans le système catalytique décrit à l'essai 8 du Tableau 1.

Tableau 3

| Temps (H) | Ligand | Sélectivité[a] Cet. | Ald. | Acide (%) | Rdt Acide isolé (%) | Rdt/$O_2$ (%) |
|---|---|---|---|---|---|---|
| 7 | Pyridine | 8 | 21 | 71 | 64 | 65 |
| 6 | 3.5 lutidine | 8.5 | 3 | 88.5 | 73.5 | 73 |
| 5 | 3 picoline | 8 | 4 | 88 | 72.8 | 73 |
| 5 | 4 picoline | 9 | 4 | 87 | 72.4 | 70 |
| 5.5 | 4 tbupy | 10 | 3 | 87 | 76.8 | 72 |
| 2.5 | Py N-Oxyde | 3 | 60 | 37 | 42 | 45 |
| 21 | Bipyridine[b] | 6 | 2 | 92 | 84 | 80 |

a : Analyses du brut réactionnel (R.M.N.)

b : solvant Fréon 11 (autres essais Fréon 113)

## II APPLICATION DU PROCEDE A DIFFERENTS ALDEHYDES

Les résultats obtenus en fonction des conditions utilisées sont rapportées dans le tableau 4 ci-après.

TABLEAU 4

| Aldéhyde | temps (H) | Solvant | Sélectivité Ald. | Acide (%) | Autre | Rdt acide isolé (%) |
|---|---|---|---|---|---|---|
| (4-isobutylphényl)-2-propionaldéhyde | 6,5 | Fréon 11 | 1 | 96 | 3[a] | 70 |
| (4-méthylphényl)-2-propionaldéhyde | 5 | Fréon 11 | - | 97 | 3[b] | 83 |
| Phényl-3-butyraldéhyde | 0,75 | Fréon 113 | 15 | 75 | - | 67 |
| Benzaldéhyde | 0,75 | Fréon 11 | 1 | 99 | - | 80 |
| Octanal | 0,5 | Fréon 113 | 1 | 99 | - | 99 |
| Crotonaldéhyde | 2 | Fréon 11 | 28 | 66 | 6[c] | 53 |

a : (4-isobutylphényl)méthylcétone
b : (4-méthylphényl)méthylcétone
c : Acide acétique

L'examen de ce tableau montre que des dérivés substitués de l'hydratropaldéhyde sont oxydés en acides avec de bons rendements (70-83%). Les dérivés aromatiques tels que le benzaldéhyde sont rapidement oxydés (45 min.). Les aldéhydes insaturés, connus pour être difficiles à oxyder peuvent être transformés en acide (ex. : crotonaldéhyde). Enfin, les aldéhydes aliphatiques sont très facilement oxydés (cas de l'octanal)

**Claims**

1. Procédé d'oxydation de composés organiques à l'aide de dioxygène, caractérisé en ce qu'on soumet à l'action de l'oxygène moléculaire un composé organique oxydable, en opérant dans un solvant exempt d'alcool, en présence d'un système catalytique comprenant un sel métallique et, le cas échéant, un composé capable de faciliter la solubilisation du sel métallique.

2. Procédé selon la revendication 1, caractérisé en ce qu'on opère à des températures comprises entre + 20°C et - 50°C, de préférence comprises entre 0° et - 20°C.

3. Procédé selon les revendications 1 et 2, caractérisé en ce que le solvant mis en oeuvre est un hydrocarbure halogéné, en particulier un hydrocarbure partiellement ou totalement substitué par du chlore et/ou du fluor, tel que le trichloro-1,1,2 trifluoro-1,2,2 éthane et le trichlorofluorométhane.

4. Procédé selon l'une des revendications 1 à 3, caractérisé en ce que le système catalytique mis en oeuvre comprend un complexe ou un sel métallique avantageusement constitué par un sel de métal de transition, en particulier un sel de manganèse ou de cobalt.

5. Procédé selon la revendication 4, caractérisé en ce que le système catalytique comporte une base hétérocyclique azotée, soluble dans le solvant utilisé, aux températures mises en oeuvre, capable de coordiner le métal de transition, en particulier de la pyridine.

6. Procédé selon l'une quelconque des revendications 1 à 5, caractérisé en ce que le rapport des concentrations [catalyseurs] / [substrat à oxyder] est de l'ordre de 1 % à 1 o/oo préférentiellement de 1 à 0,5 % et le rapport de concentration [hétérocycle] / [métal] est de l'ordre de 5 à 50 et de préférence comprise entre 10 et 30.

7. Procédé selon l'une des revendications 1 à 6, caractérisé en ce que ledit solvant ne présente pas de point éclair.

8. Application du procédé selon l'une quelconque des revendications 1 à 7 à l'obtention d'acides carboxyliques à partir d'aldéhydes.